# EUROPEAN PATENT SPECIFICATION

(11) **EP 2 440 197 B1**
(45) Date of publication and mention of the grant of the patent: **14.08.2019**
(21) Application number: 09786705.5
(22) Date of filing: 08.06.2009
(51) Int. Cl.: A61K 31/28, A61K 31/19, A61P 35/00, A61P 25/00, A61P 25/08, A61P 25/10, A61P 25/18, A61P 25/28, A61P 29/00, A61K 31/30, A61K 31/315, A61K 31/4745, A61K 47/54, C07F 3/06, C07F 3/02, C07F 1/08, C07C 53/128

(54) **THERAPEUTICAL USE OF TERNARY COMPLEXES OF VALPROIC ACID**
THERAPEUTISCHE ANWENDUNG VON TERNÄREN VALPROINSÄUREKOMPLEXEN
UTILISATION THÉRAPEUTIQUE DE COMPLEXES TERNAIRES D'ACIDE VALPROÏQUE

(43) Date of publication of application: 18.04.2012
(73) Proprietor: Université Paris-Sud XI, 91405 Orsay Cedex (FR); Centre National de la Recherche Scientifique (C.N.R.S.), 75794 Paris Cedex 16 (FR); Sylla-Iy Arreta Veitia, Maite, 75003 Paris (FR)
(72) Inventor: SYLLA-IY ARRETA VEITIA, Maite, F-75003 Paris (FR); DUMAS, Françoise, F-92350 Le Plessis Robinson (FR); MORGANT, Georges, F-94270 Le Kremlin Bicetre (FR)
(74) Representative: Grosset-Fournier, Chantal Catherine
(86) International application number: PCT/IB2009/053229
(87) International publication number: WO 2010/143020

(56) References cited:
- ABUHIJLEH A LATIF: "Ternary copper(II) complexes of the anticonvulsant drug valproate with diimines as superoxide dismutase mimics" JOURNAL OF INORGANIC BIOCHEMISTRY, vol. 68, no. 3, 1997, pages 167-175, XP007911040 ISSN: 0162-0134
- SYLLA-IYARRETA VEITIA M ET AL: "Synthesis, structural analysis and anticonvulsant activity of a ternary Cu(II) mononuclear complex containing 1,10-phenanthroline and the leading antiepileptic drug valproic acid" BIOCHIMIE, MASSON, PARIS, FR, vol. 91, no. 10, 1 October 2009 (2009-10-01), pages 1286-1293, XP026583909 ISSN: 0300-9084 [retrieved on 2009-06-27]
- LEMOINE P ET AL: "Synthesis, crystal structures, and anti-convulsant activities of ternary [Zn<II>(3,5-diisopropylsalicylate)2], [Zn<II>(salicylate)2] and [Zn<II>(aspirinate)2] complexes" JOURNAL OF INORGANIC BIOCHEMISTRY,, vol. 98, no. 11, 1 November 2004 (2004-11-01), pages 1734-1749, XP004619816 ISSN: 0162-0134
- DATABASE CA [Online] CHEMICAL ABSTRACTS SERVICE, COLUMBUS, OHIO, US; October 2000 (2000-10), CANGER, RAFFAELE ET AL: "Clinical efficacy and tolerability of magnesium valproate as monotherapy in patients with generalized or partial epilepsy : A non-blinded study of 320 patients" XP002563944 retrieved from STN Database accession no. 2000:804977

## Description

The present invention relates to ternary complexes of valproic acid and to their therapeutical use.

Copper is an essential element in human normal metabolism. Copper, as essential fats, amino acids and enzyme cofactors, is required for normal metabolism of all tissues. Coordinate forms of copper are more stable than the corresponding ionic species, therefore, in biological systems, copper exists as a variety of complexes. Moreover, apart Wilson's disease, there is no human chronic degenerative diseases known to result from a non industrial exposure to copper. On these grounds, the syntheses and antiarthritic activity of copper complexes of non-steroidal anti-inflammatory compounds such as aspirin and derivatives and niflumic acid have been studied (J. R. Sorenson, Copper chelates as possible active forms of the antiarthritic agents, (J. Med. Chem. 19 (1976) 135-148). These complexes have been found to be more active and desirable drugs that their parent ligands themselves, suggesting that the activity of such metal-based drugs may be due to the *in vivo* formation of metallic complexes.

Valproic acid, the common name of 2-propylpentanoic acid, (VPA) and its sodium salt (Valp) have shown a wide spectrum of activity as anticonvulsant drugs (A. G. Chapman, P. E. Keane, B. S. Meldrum, J. Simiand, J. C. Vernieres, Mechanism of anticonvulsant action of valproate, Prog. Neurobiol. 19 (1982) 315-359) and is well established as first-line antiepileptic agent.

Chemically, VPA is a short, C8 branched fatty acid. It is widely used as the first line drug in the treatment of epileptic patients with generalized and partial seizures and bipolar disorder. It is an anticonvulsant indicated for use as monotherapy and adjunctive therapy in the treatment of simple or complex absence seizures. Indication of VPA has extended to psychiatric disorders, for example in the treatment of manic episodes associated with bipolar disorder, migraine prophylaxis, management of trigeminal or post herpetic neuralgia, management of diabete neuropathy, treatment for alcohol withdrawal and dependence and reduction of cocaine use (Peterson, G. M., Nauton, M.., Valproate: a simple chemical with much to offer, J. Clin. Pharm. Ther. 2005, 37 (suppl 2), 25-42; C. Rosenberg, The mechanism of action of valproate in neuropsychiatric disorders: can we see the forest for the trees? Cell Mol. Life Sci., 64 (2007) 2090-2103). VPA is well tolerated in most patients and has an impressive safety profile.
VPA increases gamma-amino butyric acid levels in the central nervous system and is a strong inhibitor of the enzyme histone deacetylase 1 (HDAC1) inducing tumour cell differentiation, apoptosis, and growth arrest with application in cancer therapy [M. Kostrouchova, Z. Kostrouch, M. Kostrouchova, Valproate, a molecular lead to multiple regulatory pathways, Folia Biol. (Praha) 53 (2007) 37-49; S.L. Stapleton, P.A. Thomson, C.N. Ou, S.L. Berg, L. McGuffey, B. Gibson, S.M. Bianey, Plasma and cerebrospinal fluid pharmacokinetics of valproate after oral administration in non human-primates, Cancer Chemother. Pharmacol. 61 (2008) 647-652]. Histone deacetylase (HDAC) inhibitors are an emerging class of therapeutic agents demonstrating significant efficacy and safety in pre-clinical and early clinical studies. In recent years a number of novel and structurally diverse HDAC inhibitors have been identified as effective anti-tumor agents and are doing well in late-stage clinical trials. Studying the signaling pathways that govern the mode-of-action of HDAC inhibitors and their functional implications in the cell is now an area of intense research. In vivo as single agent or in combination with differentiating agents, VPA has shown clinical activity in patients with myelodisplasic syndrome (MDS):[Kuendgen, A.; Strupp, C.; Aivado, M.; Bernhardt, A.; Hildebrandt, B.; Haas, R.; Germing, U.; Gattermann, N.; Treatment of myelodysplastic syndromes with valproic acid alone or in combination with all-trans retinoic acid, Blood, 2004, 104, 1266].

HDAC1 is needed for HIV'gene to remain hidden in the infected cells DNA. VPA can stimulate also the release of virus from latently infected CD4⁺ T-cells *in vitro.* It may be useful in the treatment of HIV infection by reducing the number of dormant infected T cells, making the virus more accessible to attack by other antiretrovirals. VPA could also potentiate the efficacy of chemotherapy for EBV-positive tumor patients. There is compelling reason to believe that HDACIs may be a promising class of compounds for treating endometriosis and ademomyosis as well: (Liu X, Guo SW Fertility and Sterility, 2008. A pilot study on the off-label use of valproic acid to treat adenomyosis. Fertil Steril 2008;89:246-50).

VPA was also shown to promote neuronal differentiation in association with HDACi [I. Tag Yu,; Park, S. H. Kim, J.-S. Lee, Y.S. Kim, H. Son, Neuropharmacol 56 (2009) 473-480] and induction of pluripotent stem cells from primary human fibroblasts with only two factors, suggesting the possibility of reprogramming through purely chemical means, which would make therapeutic use of reprogrammed cells safer and more practical [D. Huangfu, K. Osafune, R. Maehr, W. Guo, A. Eijkelenboom, S. Chen, W. Muhlestein, D. A. Melton, Nature Biotechnology, 26 (2008) 1269-1273].

Along with the desired effects, VPA therapy can induce side effects such as dyspepsia, obesity and important endocrine dysfunctions (F. Dutheil, P. Beaune, M.-A. Loriot, Xenobiotic metabolizing enzymes in the central nervous system: Contribution of cytochrome P450 enzymes in normal and pathological human brain, Biochimie, 90 (2008) 426-436; A. Verrotti, R. Greco, G. Latini, F. Chiarelli. Endocrine and metabolic changes in epileptic patients receiving valproic acid, J. Pediatr. Endocrinol. Metab. 18 (2005) 423-430).

It has been demonstrated that it can cause hepatotoxicity and teratogenicity (E. Sobol, M. Bialer, B. Yagen, Tetramethylcyclopropyl analogue of a leading antiepileptic drug, valproic acid. Synthesis and evaluation of anticonvulsant activity of its amide derivatives, J. Med. Chem. 47 (2004) 4316-4326; R. A. Blaheta , H. Nau , M. Michaelis, J. J. Cinatl, Valproate and valproate-analogues: potent tools to fight against cancer, Curr Med Chem. 9 (2002) 1417-1433. Less common side effects are pancreatitis, dizziness, drowsiness, hair-loss, headaches, nausea, sedation and tremors (M. L. Houben, I. Wilting, H. Stroink, P. J. van Dijken, Pancreatitis, complicated by a pancreatic pseudocyst associated with the use of valproic acid, Eur J Paediatr Neurol. 9 (2005) 77- 80; K. S. Walia, E. A. Khan, D. H. Ko, S. S. Raza, Y. N. Khan, Side effects of antiepileptics-a review, Pain Practice 4 (2004) 194-203.

Therefore, VPA analogs or derivatives (M. K. Trojnar; E.Wierzchowska-Cioch, M. Krzyzanowski, M. Jargiello, S. J. Czuczwar, New generation of valproic acid, Polish J. Pharmacol. 56 (2004) 283-288) and formulations (M. Bialer ,A. Haj-Yehia, K. Badir, S. Hadad, Can we develop improved derivatives of valproic acid?, Pharm. World Sci. 16 (1994) 2-6) have been developed in an effort to overcome undesired effects.
Some authors have demonstrated that metal salts of physiological importance can bind to sodium valproate leading to the formation of compounds presenting lower solubility than sodium valproate itself. Such interaction may have effects upon the body functioning (Healy M.A. Aslam M. Journal of clinical and Hospital Pharmacy (1986), 11, 189-198.) in particular concerning the rare but existing side effects (hair loss for example). Serum trace metal homeostasis of Zn and Cu might be affected by VPA therapy, but not the convulsive disorder itself (Armutcu, F.; Ozerol, E.; Gurel, A.; Kanter, M.; Vural, H.; Yakinci, C.; Akyol, O.; Biological Trace Element Research, 2004, 102, 1-10) and after one year of therapy, VPA does not affect levels of Cu, Zn, Se, GSH-PX and CuZnSOD concentrations in epileptic children's serum (Verrotti, A.; Basciani, F.; Trotta, D.; Pomillio, M.P.; Morgese, G. ; Chirelli, F., Epilepsy research, 2002, 48, 71-75) whereas. Finally for some authors VPA treatment decreases the plasma and red blood cell zinc levels but the mechanism is still obscure.

Although sodium valproate is a very effective drug, since it is administered repetitively as chronic treatment, the adverse effects associated with antiepileptic therapy are of a major concern. Valproic acid as others antiepileptic drug is associated with certain rare but severe side effects such as teratogenicity has considerable adverse effects including the potential for fatal hepatotoxicity.

The inventors have surprisingly found that metal-based complexes of valproic acid sodium salt (Valp) with nitrogen donor ligands, show very interesting pharmacological property and could lead to less side-effect. Indeed the 1,10-phenanthroline (phen) and its derivatives are well known to exhibit antifungal, antiviral and antimycoplasmal activities. Their cytotoxicity might be due to chelation of transition metals such as copper or iron for example in test media. Complexation with some divalent transition metals enhances these biological activities. Thus, antiviral activity was found for divalent transition metals chelates with substituted phenanthrolines. Moreover, such complexes are used as DNA intercalating agents and have been found useful for examining distinctive conformations along the DNA helix. Ternary complexes of Cu(II) with 3,5-diisopropylsalicylate and substituted phenanthrolines have been synthesized, and among them, the most potent proved to be bis(diisopropylsalicylato)(2,9-dimethylphenanthroline)copper(II) which exhibits cytotoxicity comparable with cisplatin [PtCl₂(NH₃)₂], an anticancer drug (J. D. Ranford, P. J. Salder, D. A. Tocher, J. Chem. Soc. Dalton Trans. (1993) 3393-3399.). Recently, ternary mononuclear and binuclear copper(II) complexes of 1,10-phenantroline and salicylate ligands as well as ternary zinc(II) complexes of 1,10-phenantroline with 3,5-diisopropylsalicylate, salicylate and aspirinate have been synthesized, characterized and evaluated for their anti-convulsant activities. This is disclosed in P. Lemoine et al., J. Inorg. Biochem., vol. 98, no. 11, 2004, pages 1734-1749. Ternary copper(II) complexes of valproate with diimines such as Cu(VAL)2(PHEN) are disclosed in A.Latif Abuhijleh, J. Inorg. Biochem., vol. 68, no. 3, 1997, pages 167-175.

Consequently an object of the present invention is a metal-based ternary complex of valproic acid with nitrogen donor ligands, in particular with diimines or diamines, wherein the metal in the complex is selected from the group consisting of Mg(II) and Zn(II) for its use in the treatment of a condition responsive to valproic acid therapy selected from cancer and inflammatory diseases.

The invention is fully defined by claims 1-5.

In an aspect, the responsive condition is a neuroaffective disorder selected from the group comprising seizures, epilepsy, bipolar disease, schizophrenia, mood disorders, affective disorders, neuropathic pain, migraine headaches and neurodegenerative syndromes.

In another advantageous embodiment according to the invention, the responsive condition is selected from cancer such as cervical, prostate, epithelioid mesothelioma, ovarian, leukemia/lymphoma and brain tumors like for example neuroblastoma and inflammatory disease such as Rheumatoid arthritis, shoulder tendinitis or bursitis, Gouty arthritis, Polymyalgia rheumatica, osteoarthritis, fibromyalgia, muscular low back pain and muscular neck pain.

According to the invention, the diimine may be selected from the group comprising 1,10-phenantroline, pyridine, imidazole,1-methylimidazole, 2,9-dimethyl-1,10-phenantroline, phendione, 2,2'-bipyridine, 2,2'-bisbenzimidazole, thiabendazole (2-(4'-thiazolyl)-benzimidazole), 2-(4,5-dihydroxyoxazolin-2-yl)-1-H-benzimidazole, 2-(2-pyridyl)-benzimidazole, 2-pyridylamine, 2-amino-2-thiazoline, nicotinamide, 2-picolinamide, 3-picoline, 4-picoline, dimethyldipicolinate.

According to the invention the diamine may be selected in the group comprising ethylenediamine, 1,3-diaminopropane, N-methyletlylenediamine, *N*,*N*'-dimethylethylenediamine and derivatives.

According to an advantageous embodiment of the instant invention, the metal in the complex is selected from the group comprising Mg(II) and Zn(II).

In a particularly advantageous embodiment of the invention, the metal-based ternary complex of valproic acid with diimines is selected from the group comprising Mg(VALP)₂PHEN and Zn(VALP)₂PHEN.

An object of the invention is also a metal-based ternary complex of valproic acid with diimines wherein the metal in the complex is selected from the group consisting of Mg(II) and Zn(II), in particular Mg(VALP)₂PHEN and Zn(VALP)₂PHEN.

A further object of the invention is also a metal-based ternary complex of valproic acid with diamines wherein the metal in the complex is selected from the group consisting of Mg(II) and Zn(II); in an advantageous embodiment of said complex, the diamine selected in the group comprising ethylenediamine, 1,3-diaminopropane, N-methyletlylenediamine, *N*,*N*'-dimethylethylene-diamine and derivatives.

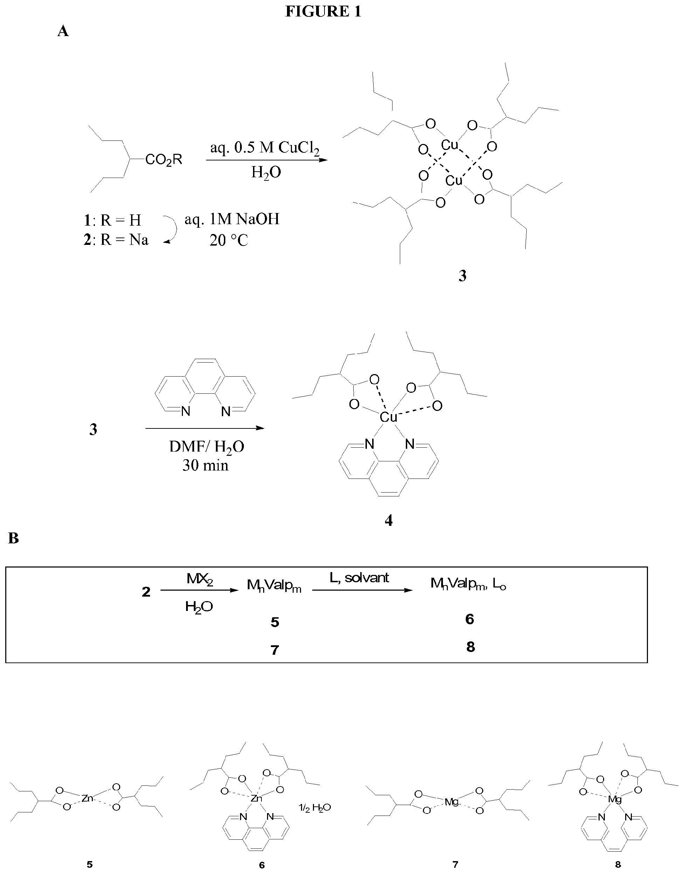

The metal-based ternary complex of valproic acid according to the invention may be synthesized by any methods known from the one skilled in the art, for example according to Scheme 1 in figure 1B.

Valproic acid (VPA) or its sodium salt (Valp) is reacted in a basic aqueous solution with a compound of formula M(X)₂ or M(OH)₂ wherein X represents an halogen atom selected from chloride, bromide, iodide and fluoride and M is a metal selected from Cu(II), Mg(II), Zn(II), Co(II), Se(II), and Mn(II) to give a metal-based binary complex MₙValpₘ which is reacted in an organic medium with a nitrogen donor ligand (L), for example a diimine or a diamine to give a metal-based ternary complex MₙValpₘLₒ.

Another disclosure is a metal-based ternary complex of valproic acid for use in a method for inducing a neuroprotective effect comprising administering to a subject in need thereof an effective amount of a metal-based ternary complex of valproic acid with nitrogen donor ligands, in particular with diimines or diamines.

A further disclosure is a a metal-based ternary complex of valproic acid for use in a method for treating cancer comprising administering to a subject in need thereof an effective amount of a metal-based ternary complex of valproic acid with nitrogen donor ligands, in particular with diimines or diamines.

Still another disclosure is a a metal-based ternary complex of valproic acid for use in a method for treating inflammatory diseases comprising administering to a subject in need thereof an effective amount of a metal-based ternary complex of valproic acid with nitrogen donor ligands, in particular with diimines or diamines.

Still another disclosure is a metal-based ternary complex of valproic acid with nitrogen donor ligands, in particular with diimines or diamines for use as a drug suitable for condition responsive to valproic acid therapy selected from bipolar disease, schizophrenia, mood disorders, affective disorders, neuropathic pain, migraine headaches, neurodegenerative syndromes, cancer and inflammatory disease.

Still another disclosure is a pharmaceutical composition comprising as active principle at least one metal-based ternary complex of valproic acid with nitrogen donor ligands, in particular with diimines or diamines associated with any pharmaceutical excipients for use as a drug suitable for condition responsive to valproic acid therapy selected from bipolar disease, schizophrenia, mood disorders, affective disorders, neuropathic pain, migraine headaches, neurodegenerative syndromes, cancer and inflammatory disease.

Still another disclosure is at least one metal-based ternary complex of valproic acid with nitrogen donor ligands, in particular with diimines or diamines associated with any pharmaceutical excipients for the preparation of a drug suitable for use in the treatment of a disease selected from the group comprising bipolar disease, schizophrenia, mood disorders, affective disorders, neuropathic pain, migraine headaches, neurodegenerative syndromes, cancer and inflammatory disease.

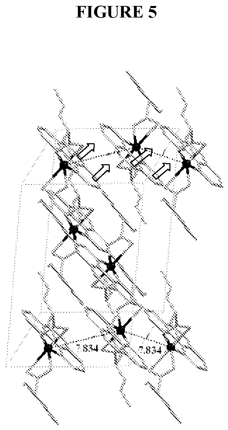

The invention is illustrated by the following example and figures 1 to 9.
Figure 1A illustrates the synthesis of the copper-based ternary complex of valproïc acid. **3** is the binary complex with copper and **4** is the ternary complex with copper (Cu(VALP)₂PHEN). Figure 1B illustrates the general procedure for synthesis of the metal-based ternary complex of valproic acid according to the invention. Sodium salt of valproic acid **2** (Valp), **5** is binary complex with zinc and **7** is binary complex with magnesium; **6** is the ternary complex with zinc (Zn(VALP)₂PHEN) and **8** the ternary complex with magnesium (Mg(VALP)₂PHEN).
Figure 2 illustrates the crystal data and structure refinement parameters for compound **4**
Figure 3 illustrates the electronic and IR spectral Data for Cu(II) complexes **3** and **4.**
Figure 4A shows a perspective view (Cameron drawing) of the crystal structure of [Cu(Valp)₂ phen] **4** and the atomic numbering scheme (symmetry code i: -x+1; y; -z-1/2) with the long Cu-O bonds of the chelating valproate anions as hollow bonds. The ellipsoids enclosed 30% probability. Figure 4B illustrates the selected bond distances (Å) and bond angles (°) for **4;** i atoms are generated by two fold rotation about symmetry code (i) Symmetry code i: -x+1, y, -z-1/2
Figure 5 represents a view of the unit cell of complex **4** showing the aromatic slipped stacking of the phen ligands, with the interaction (marked with an arrow) of two rings of each phen with the nearest molecules with a Cu˙˙˙Cu distance of 7.834 Å (copper atoms in black).
Figure 6 illustrates the EPR spectrum observed from the MeOH solution of complex **4** at 77 K.

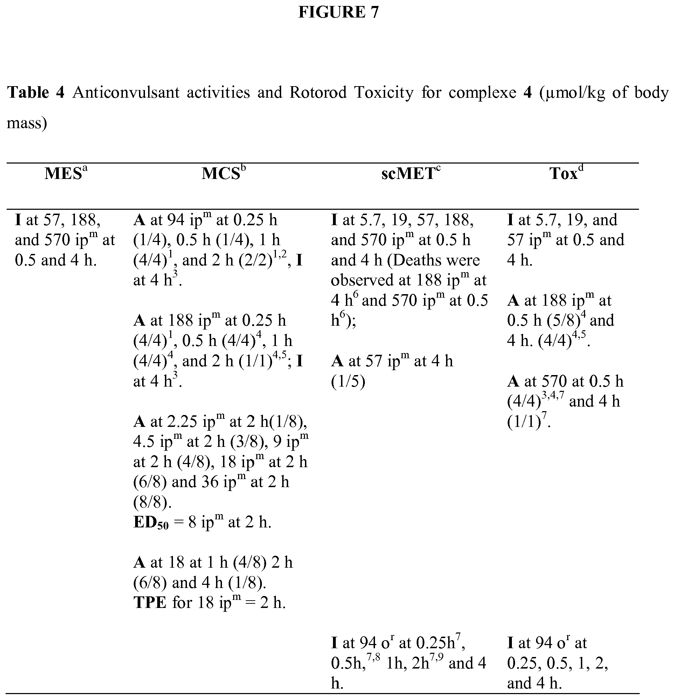

Figure 7 shows the anticonvulsant activities and Rotorod Toxicity for complex **4** (µmol/kg of body mass). ^{a} MES = Maximal Electroshock seizure test, ^{b}MCS: Psychomotor seizure or Minimal clonic seizure test, ^{c} MET = subcutaneous Metrazol seizure threshold test, ^{d} Tox: Rotorod Toxicity test. **A** = Activity at indicated dose and time of challenge were #/# is the number of effected animals/the number of animals per study which ranged from 1 to 8 animals. **I** = Inactive, ip = intraperitoneal, o = oral, sc = subcutaneous, m = mice, r = rats. ED₅₀ = Effective dose for 50 % of the treated animals. TPE = Time to Peak Effect of a treatment dose.
   ¹ Minimal motor impairment, ² One death' ³ All died before test challenge, ⁴ Unable to grasp rod, ⁵ Three deaths, ⁶ Death without seizure, ⁷ Death following continuous seizure, ⁸ Continuous seizure activity.9 tonic extension.
Figure 8 illustrates the toxicity of substances **3-8** according to the invention tested in anti-inflammatory assay in zebrafish. The molecular structures of the compounds **3-8** are shown in Figure 1.
Figure 9 illustrates the evaluation of the anti-inflammatory activity with Indomethacin used as positive control evidenced by the percentage of Relative Leukocyte Migration inhibited by the substances tested at the specified concentration (chosen as the Maximum tolerated dose determined in fig 8) in zebrafish. Results are expressed as mean ± s.e.m.

### Example: Synthesis of the copper complexes

### 1.1 Material and methods

### 1.1.1 Materials

Anhydrous copper (II) chloride 99% and 2-propylpentanoic acid (Acros, Belgium), sodium hydroxide and *N*,*N-*dimethylformamide (SDS, France), 1,10-phenanthroline (Avocado, United Kingdom) were purchased from the respective concerns and used as received. All other chemicals and solvents used were of analytical reagent grade and produced from commercial sources.

### 1.1.2. Physical measurements

The elemental analysis (C, H, and N) was performed on a Perkin-Elmer 2400 CHN analyzer. UV-vis spectra of methanol solutions were recorded on a UV-160 Shimadzu spectrophotometer. Infrared spectra were recorded using a Bruker FT/IR vector 22 spectrometer. Melting points were determined in a Tottoli type S Büchi capillary melting points apparatus and are uncorrected.

### 1.1.3 bis-(2-propylpentanoato)(1,10-phenanthroline)copper(II) [Cu(Valp)₂phen] (4) (comparative)

The synthesis is illustrated in figure 1A

### 1.1.3.1. Tetrakis-µ-2-propylpentanoato dicopper(II) [Cu₂(Valp)₄] (3)

A solution of CuCl₂ (13.44 g, 0.1 mol) in water (200 mL) was filtered and added with continuous stirring to sodium valproate, prepared by neutralizing valproic acid (14.42 g, 0.1 mol) in water (100 mL) with 1 M NaOH (100 mL) at 20 °C. A blue green powder was immediately obtained, recovered by vacuum filtration and dried over silica-gel in a dessicator for 24 h.
Yield: 90% (28.597 g).
*Anal.* Calc. for C₃₂H₆₀Cu₂O₈ (**3**): C, 53.54; H, 8.70%. Found: C, 53.38; H, 8.55%; M.p. = 293-294 (with decomposition); IR (neat, cm ⁻¹): v(C-H) 2957, v*as*(COO⁻) 1578, v*sy*(COO⁻) 1417.

### 1.1.3.2. Bis-(2-propylpentanoato)(1,10-phenanthroline)copper(II) [Cu(Valp)₂phen] (4)

1,10-Phenanthroline (1.03 g, 5.72 mmol) was added to a vigorously stirred solution of [Cu₂(Valp)₄] **3** (2.00 g, 2.78 mmol) in dimethylformamide (DMF) (140 mL). Water (20 drops) was then added and the resulting solution-suspension was stirred for 30 min and allowed to stand at room temperature. Dark green crystals, needle-like of **4** were deposited over a period of 23 days. Single crystals suitable for RX analysis were collected in the resulting suspension and dried under vacuum at 40 °C.
Yield: 45% (0.665 g).
*Anal.* Calc. for C₂₈H₃₈CuN₂O₄ (**4**): C, 63.43; H, 7.22; N, 5.28. Found: C, 63.39; H, 7.46; N, 5.46%. M.p. = 206-209. IR (neat, cm⁻¹): v(C-H) 2951, v*ₐₛ*(COO⁻) 1567, v(C=C), v*_{sy}*(COO⁻) 1394.

For a preparative aspect, a DMF solution of an equimolar mixture of phen (10.02 g, 0.556 mol) and binuclear complex **3** (20.00 g, 0.278 mol) was stirred for 30 min, filtered and evaporated to dryness under vacuum to yield pure **4** as a bluish green powder (14.42 g, 98%).

### 1.1.4. X-ray crystallographic experimental

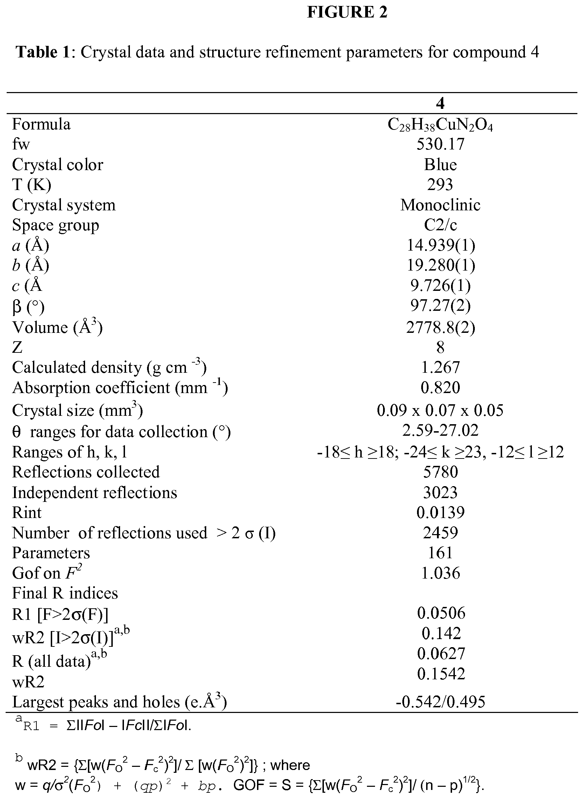

Crystal data and experimental conditions are listed in Table 1 of figure 2. Structural data for **4** was performed with monochromated Mo-Kα radiation (λ = 0.71070 Å) on a Nonius Kappa detector at 293 K, with data collection and reduction using HKL package (Z. Otwinowski, W Minor, Methods & Enzymology. Vol. 276, Macromolecular Crystallography, Part A, C. W. Carter Jr, R. M. Sweet Eds, New York: Academic Press (1997) 307-326).

A monoclinic unit cell: a = 14.939(1), b = 19.280(1), c = 9.726(1) Å, β = 97.268(5)°, V = 2778.9(2) Å³, Z = 8 and *d*(calc) = 1.267 Mg.m⁻³ yielded 5780 total reflections to a 2θₘₐₓ = 54.04°. The structure was solved by direct methods in the space group C2/c using SIR97 (A. Altomare, M. C. Burla, M. Camalli, G. L. Cascarano, G. Giacovazzo, A. Guagliardi, A. G. G. Moliterni, G. Polidori, R. Spagna, SIR97: a new tool for crystal structure determination and refinement, J. Appl. Crystallogr. 32 (1999) 115-119) and refined by least-squares methods on *F*² using SHELXL-97 (G. M. Sheldrick, SHELXL-97, Program for Crystal structure refinement, University of Göttingen, Germany, (1997)).

The crystal parameters, data collection and the refinement details of compound **4** are given in Table 1. Non-hydrogen atoms were refined with anisotropic thermal parameters. Hydrogen atoms were calculated for idealized geometries and allow riding on their parent atoms with isotropic parameter equal 1.2 times to those of the attached atoms. For all 3023 unique reflections the final anisotropic full-matrix least squares refinement on *F*² for 161 variables converged at R1 = 0.051 and wR₂ = 0.1423 with a goodness of fit (gof) of 1.036. The drawings of the molecules were realized with CAMERON (D. J. Watkin, C. K. Prout, L. J. Pearce CAMERON, Chemical Crystallography Laboratory, University of Oxford, UK (1996)).

### 1.1.5. EPR spectrometry

EPR spectra were recorded on a Bruker Elexsys 500 EPR spectrometer operating at X-band frequency (9.44 GHz) equipped with an shq0011 cavity fitted with an Oxford Instruments liquid helium cryostat. The following instrument settings were used : field modulation amplitude frequency of 100 kHz, field modulation amplitude of 0.5 mT, time constant of 0.04 s, sampling time 20 ms, 2048 sampling points, field sweep 0.15 T, microwave power of 10 mW at 80 K and 1 mW at 10 K. Progressive microwave power saturation has been performed increasing attenuation by 1 dB step from 0 to 60 dB corresponding to microwave power from 200 mW to 0.2 µW. Xsophe has been used for EPR spectra simulation of the non saturated experimental spectrum after subtraction of a solvent tube spectrum (Graeme R. Hanson, Kevin E. Gates, Christopher J. Noble, Mark Griffin, Anthony Mitchell, Simon Benson, XSophe-Sophe-XeprView®. A computer simulation software sui te (v. 1.1.3) for the analysis of continuous wave EPR spectra, J. Inorg. Biochem, 98 (2004) 903-916). The g and A values are the one obtained to reasonably fit the experimental and the derivative spectrum with the calculated one using a g and A strain line width model. In the case of a mixture of complexes in solution, the different lines of a single complex have been differentiated using the progressive microwave saturation experiments, or the equilibrium displacement.

Four type of saturated MeOH solutions of complex 4 were studied, three of which being saturated with methanolic solution of the ligands valproic acid, phenanthroline or valproic acid and phenanthroline.

### 1.1.6. Determination of anticonvulsant activities and Rotorod toxicity of [Cu Valp₂ Phen] (4) (not part of the invention)

Anticonvulsant activities were determined by the National Institutes of Health-National Institute of Neurological Disorders and Stroke-Antiepileptic Drug Development program for the detection and evaluation of compounds as anticonvulsants agents. Compounds were suspended in polyethylene glycol prior to injection into male Carworth Farms # 1 mice. In Phase I studies, identification of anti-convulsant activity, test compounds were given intraperitonealy (ip) at 57, 188 and 570 milligrams-per-kilogram (mg/kg) of body mass, and protection against Maximal Electroshock (MES), Minimal Electroshock (MCS) and/or Metrazol (scMET) induced seizures was determined 30 min and 4 h later. All statistics were obtained by Probit analysis.

### - Maximal electroshock seizure test

Maximal electroshock seizure (MES) were elicited with a 60 cycles alternating current of 50 mA, five to seven times that necessary to elicit minimal electroshock seizures, delivered for 0.2 s via corneal electrodes. A drop of 0.9% saline solution was instilled into both eyes prior to application of electrodes in order to prevent death. Abolition of the hind limb tonic extension component of seizure is defined as anticonvulsant activity.

### - Minimal electroshock or Psychomotor seizure test

This test was designed to detect compounds that are missed with the MES tests which employ a larger current of 50 mA. The delivery of a less intense electric stimulation which produces less intense stimulation of the CNS (Central Nervous System) and brain inflammation than the MES test, via corneal electrodes of 6 Hz, 32 mA or slightly higher current, for 3 s, is used to produce psychomotor seizures, following instillation of a drop of 0.9% saline solution prior to this application of electric current. Abolition of the visual stereotypic behavior characterized as minimal automatist behaviors, similar to the aura that human patients experience during psychomotor seizure, is defined as antipsychomotor seizure activity.

### - Subcutaneous (s.c) pentylenetetrazol (Metrazol) seizure threshold test

A dose of Pentylenetetrazol, a potent CNS stimulant, which produces seizures in greater than 97% of treated mice, at 85 mg/kg of body mass, was administered s.c. as a 0.5% saline solution at the posterior neck midline. Animals were observed for 30 min. Failure to observe even a threshold seizure, a single episode of clonic spasms of at least five second duration, is defined as anticonvulsant activity.

### - The Rotating Rod test (Tox)

This qualitative test was used to evaluate 'neurotoxicity', central nervous system stimulation or depression. Depression of CNS activity is a common feature of all antiepileptic drugs. Mice or rats are placed on a 1 inch diameter knurled plastic rod rotating at 6 revolutions per min. Normal mice and rats can remain on a rod rotating at this speed indefinitely. Neurologic toxicity, either CNS stimulation or depression, is evidenced by an inability to grasp the rotating rod of the mouse or rat to remain on the rotating rod for 1 min. It may be attributed to CNS depression due to sedative and/or hypnotic activities, or a state of eminent death, due to medullary paralysis at high doses, a common unwanted side effect caused by high doses of all anticonvulsant drugs.

### 1.1.7. Determination of anti-inflammatory activities and toxicity (maximum tolerated dose) of metal chelates (3) - (8)

### - In vivo anti-inflammatory efficacy studies by using zebrafish

All chemicals were firstly dissolved in dimethylsulfoxide, DMSO 99.5% (GC) Sigma-Aldrich), and then diluted in embryo medium E3 (5 mM NaCl, 0.17mM KCl, 0.33 mM CaCl₂, 0.33 mM Mg₂SO₄, 10-5% methylene blue). The final concentration of DMSO did not exceed 0.1% which caused no damage to the animals.

During the course of the experiments, transgenic zebrafish of the *fli*-1: EGFP lines were used. Once the fertilized eggs were collected, they were reared in embryo medium E3 in an incubator at 28 °C. The 20 hours post fertilization (hpf), 1-phenyl-2-thiourea (PTU) (Sigma-10×(0.03%)) was added to larvae's medium in 1:9 proportion to prevent the formation of melanophores in zebrafish embryos and larvae [Karlsson, J., von Hofsten, J. and Olsson, P. Mar. Biotechnol., 2001, 3, 522-527].

### - Toxicological evaluation

The Maximum Tolerated Concentration (MTC) was defined as the maximum concentration at which no death or signs of toxicity (in the circulatory system)were observed. The MTC in the fish water was initially determined by incubating zebrafish larvae resulting from 4 days post fertilization (dpf) with compounds assayed in E3. During 24 hours, 5 larvae of zebrafish per well were placed in a tissue culture in 24 well plates using tested compounds at 10 µM, 30 µM and 100 µM concentration in the medium. Following the 24 h period of incubation, the characteristics of the zebrafish larvae were examined. The MTC were determined in treated groups at 5dpf after 24 h exposure to the tested drugs.

### - Anti-inflammatory experimental procedure: Tail section

The experimental corroboration of anti-inflammatory activity was done by using a new *in vivo* test development using zebrafish biological material [Siverio-Mota, D., de Witte, P. *et al,* 2009, *in preparation*]*.* The principal aspects of this new biological assay are the following. First, ten larvae from 4 dpf were used per treated and control groups. In each case, the MTC was used as the assay concentration and indomethacin (Merck Sharp & Dohme) at 30 µM was used as control group. These larvae were placed in wells of a 24 wells tissue culture plate at assay's concentrations in 1mL final volume. After one hour, the larvae were anesthetized by immersion in E3 containing tricaine (Westerfield, M., The Zebrafish Book: A Guide for the Laboratory Use of Zebrafish (Brachydanio rerio). 1995, Eugene, OR, University of Oregon Press), the complete section of the tail was performed with a sterile scalpel. Once tails were cut, zebrafish were placed again in a 24 wells tissue culture plate at assay's concentrations together with 10 µg/mL solution of lipopolysaccharide to stimulate the leukocyte migration. The temperature of incubation was kept from 20 to 22 °C. After 7 h, the larvae were fixed in 4% paraformaldehyde for 5 minutes then washed twice with PBS. One milliliter of Leucognost Pot® staining solution was added and 10 minutes later, each zebrafish was observed with a microscope and the leukocyte migration analyzed [Siverio-Mota, D., de Witte, P. *et al,* 2009, *in preparation*]*.* Each assay was-performed in triplicate.

### 1.2. Results

### 1.2.1. Synthesis

As shown in scheme 1 of figure 1A, binuclear copper valproate **3** was obtained via the reaction of CuCl₂ with the sodium salt of valproic acid in the molar radio 1:1 in water. The complex **(4)** was achieved by treatment of a copper complex Cu₂Valp₄ with 1,10-phenanthroline in the molar radio 1:2 at room temperature in DMF. Dark green crystal, needle-like of [Cu(Valp)₂ phen] **4** suitable for X-ray analysis were deposited over a period of 14 to 23 days at 20 °C in 23% to 45% yield. Both complexes are soluble in ethanol, tetrahydrofuran, methanol and dimethylsulfoxide, less soluble in acetonitrile and acetone and few soluble in water, chloroform and dichloromethane.

Elemental analysis values were in good agreement within 0.3% for both complexes which showed to be a 1:2 Cu:Valp assembly for complex **3** and a 1:2:1 Cu:Valp:phen one for complex **4.** The melting point values were 293-294 °C for the complex **3** and 206-208 °C for the complex **4.**

### 1.2.2. Spectroscopic results

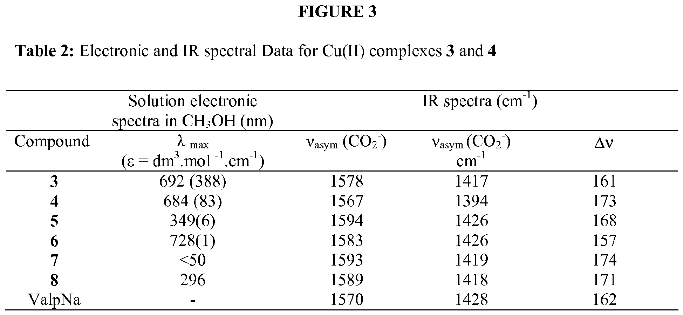

They are given in table 2 of figure 3.

The electronic UV-Visible spectra obtained in methanol solutions (1.2 10⁻³ M) of chelates **3** and **4** exhibit one very broad absorption band in the 680-690 nm region (Table 2). This band is assigned to the copper (II) *d-d* transitions. These results are according with the literature (A. L. Abuhijleh and C.Woods, Synthesis, characterization, and oxidase activities of copper(II) complexes of the anticonvulsant drug valproate, J. Inorg. Biochem. 64 (1996) 55-67).

The frequencies (cm⁻¹) of the most relevant absorption bands in the IR spectra of the studied compounds and their assignments (antisymmetric stretch, v_{asym}(CO₂) and symmetric stretch, v_{sym}(CO₂) of the valproate carboxylate group) presented in Table 2 parallel literature data. Broad bands assigned to v_{asym}(CO₂) occur between 1567 and 1578 cm⁻¹, and bands for v_{sym}(CO₂) between 1394 and 1417 cm⁻¹. The bands positions and the separation Δv [v_{asym}(CO₂) - v_{sym}(CO₂)], between 161 and 173 cm⁻¹, when compared with those of sodium valproate [v_{asym}(CO₂) 1570; v_{sym}(CO₂) 1417; Δv = 153 cm⁻¹] are in the range expected for carboxylate groups that act as unsymmetrical bidentate ligands. These parameters are comparable to those reported for mononuclear copper(II) carboxylate complexes that contain imidazoles having the CuN₂O₂˙˙˙˙˙O₂ chromophore (A. L. Abuhijleh previously cited).

Similar characteristics are shown for compounds 5, 6, 7 and 8.

### 1.2.3. Crystallographic studies of bis(2-propylpentanoate)(1,10-phenanthroline)copper(II) [Cu(Valp)₂phen](4) (not part of the invention)

The structure of [Cu(Valp)₂ phen] complex **4** has already been proposed on the basis of elemental analysis, infrared and ultraviolet spectrometry (A. L. Abuhijleh previously cited) although the crystal structure had not yet been resolved.

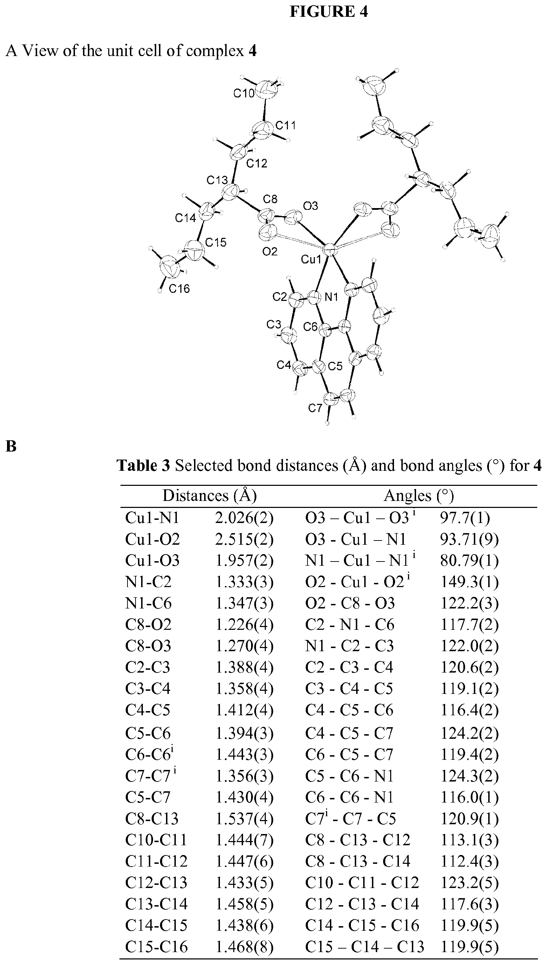

Reaction of complex **3** with phenanthroline in DMF furnished a green crystalline product in 23 to 45% yield upon standing for two or three weeks at 20 °C, which elemental analysis showed to be a 1 : 2 : 1 Cu : Valp : Phen complex. In order to determine unambiguously the structure of this compound, a single X-ray structure analysis was carried out. The molecular structure of the ternary mononuclear complex **4** is represented in Figure 4A and selected bond lengths and angles are given in Table 3 of figure 4B.

The complex crystallizes in the C2/c space group with eight molecules of **4** in the unit. The structure of **4** consists of monomeric units with the N₂O₄ donor set. The Cu atom lies on a crystallographic two fold axis which bisects the phen ligand. The asymmetric unit consists of half of the Cu(II) complex. The Cu atom exhibits a highly distorted octahedral geometry in the CuN₂O₂ + O₂ chromophore. The equatorial plane P1 (O3, O3ⁱ, N1, N1ⁱ) is formed by two oxygen atoms O3 and O3ⁱ (i = -x + 1, y, -z -1/2) from the carboxylate groups of valproate anions (Cu(II) - O3: 1.957(2) Å) and two nitrogen atoms N1 and N1ⁱ from phenanthroline ligand (Cu - N1: 2.026(2) Å). Plane P1 is twisted toward a tetrahedron with a tetrahedral twist angle *Td* of 26.6° [45]. The apical positions are occupied by the second carboxylate oxygen atoms O2 and O2ⁱ which are less tighted bonded to Cu(II) with a contact distance of 2.515 (2) Å and an O2 - Cu(II) - O2ⁱ angle of 149.3(3)°. This deformation is also evidenced by the sum of the angles N1-Cu-N1ⁱ + N1ⁱ-Cu-O3ⁱ + O3ⁱ-Cu-O3 + O3-Cu-N1 (365.9°) which is significatively different from 360°, the value expected for a square planar complex. The deviation of Cu(II) from the mean plane P2 (C13, C8, O2, O3) of the carboxylate group, which exhibits an asymmetric chelating mode, is 0.008(5) Å. The analysis of the C8-O2 and C8-O3 bond distances, 1.226(4) and 1.270(4) Å, respectively, shows that the covalent character of the Cu-O linkage is relatively large, ca 28% on the basis of the theory by Hocking and Hambley [49], even though a weak interaction exists to the second oxygen atom of the valproato ligands. The O2-C8 distance, significantly shorter than the O3-C8 one, shows that the carboxylate group is not a completely delocalized system with more keto character in the O2-C8 linkage in accord with its weaker bond to Cu.

### 1.2.4. EPR analysis of bis(2-propylpentanoate)(1,10-phenanthroline)copper(II) [Cu(Valp)₂ phen] 4 (not part of the invention)

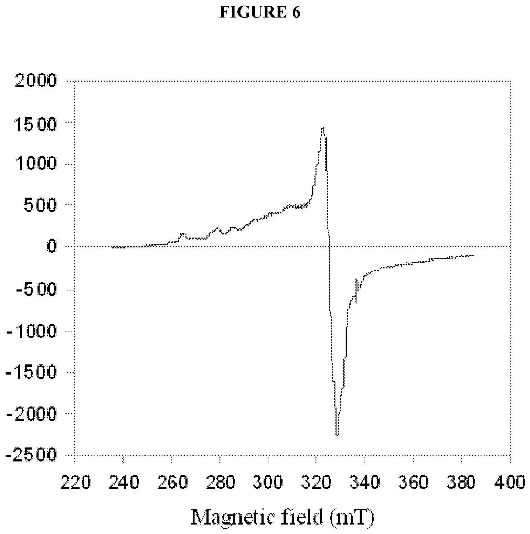

The results are given in figure 6.

The structure of the pharmacologically active anticonvulsant copper species is still a matter of controversy. In order to study the relationship between the biological activity of metallo-complex **4** and its structure in solution, EPR has been used to check the copper complex geometry and environment. Saturated solution of *[Cu(Valp)₂ phen]* **4** exhibits a mixture of different complexes which we can differentiate and identify by two sets of experiments: equilibrium displacement using separately the two ligands of the complex and a progressive micro wave saturation EPR experiment.

We have performed micro-wave power saturation on three solution of *[Cu(Valp)₂ phen]* **4** *([Cu(Valp)₂ phen]* **4** in solution, **4** in solution in the presence of an excess of valproic acid, **4** in solution in the presence of an excess of phenanthroline) from those we can postulate the existence of two different octahedral copper complexes in methanol solution of *[Cu(Valp)₂ phen]* **4:**
- a highly distorted octahedral complex (g = 2.4319, A = 110 Gss, g = 2.08) in an oxygen environment of the copper with valproic acid as ligand representing approximately 1/6 of the copper in solution,
- a distorted octahedral copper complex (g = 2.40, A = 133 Gss, g = 2.07) which is certainly the *[Cu(Valp)₂ phen],* complex which has been crystallized representing 5/6 of the copper content,

As soon as the phenanthroline concentration increases, one can obtain some triphenanthroline complex (A = 158 Gss g = 2.28, g = 2.07) which is not present in the *[Cu(Valp)₂ phen]* MeOH solution.

One can postulate on these grounds that the reported biological activity of metallo-complex **4** can be related to such mononuclear octahedral complexes containing at least valproate as ligand. However, one cannot exclude that ligand exchange to form more stable and lipophilic chelates in vivo may also play a role in producing the anticonvulsant effects.

### 1.2.5. Anticonvulsant activity of bis(2-propylpentanoate)(1,10-phenanthroline)copper(II) (not part of the invention)

The results are presented in Table 4 of figure 7. Data in this table are a thorough and detailed presentation of all of the Phase I, II, or III test results obtained at specific times of seizure induction or Rotorod Toxicity challenge, after treatment with the test metallo-complex as provided by the Antiepileptic Drug Development screening program. Phase I results are obtained by testing doses of 30, 100, and 300 mg/kg with the highest doses intended to detect activity of weakly active compounds. The most favourable outcomes of these studies are *activities* in preventing seizures caused Maximal Electroshock, Minimal Clonic Electroshock, or an injection of Metrazol, a central nervous system (CNS) stimulant as well as *inactivity* in the Rotorod Toxicity tests. Since high doses used in these initial evaluations are very high doses it is sometimes found that these doses cause undesirable toxicities. In the event that activity is found at any dose, Phase II and III studies are conducted at lower doses and alternative challenge times to quantify potentially effectiveness and useful anticonvulsant activity. All test results have been converted to µmol/kg to enable comparisons based upon relative molecular effectiveness.

As shown in Table 4, compound **4** was found to be inactive in ip treated mice given 57, 188, or 570 µmol/kg and challenged with MES at 0.5 and 4 h after treatment. Compound **4** protected mice against the Minimal Clonic Seizure following ip treatment with 94 µmol/kg prior to challenge with minimal electroshock at 0.25, 0.5, 1, and 2 h after treatment. Minimal motor impairment was observed in most mice, however two deaths occurred after the 2 h challenge. All treated mice died prior to challenge at 4 h. These results suggest that this dose of compound **4** had a rapid onset and prolonged duration of activity.

Compound **4** was also found to be active at 188 µmol/kg at the 0.25, 0.5, 1, and 2 h challenge times with an inability to grasp the rotating rod. However this dose of **4** caused three deaths at the 2 h challenge time and all mice died before the 4 h challenge consistent with a prolonged duration of action. Smaller doses of 2.25, 4.5, 9, 18, and 36 µmol/kg of **4** produced a dose-related increase in protection against the Minimal Clonic seizure when challenged at 2 h suggesting very potent activity, with the absence of toxicity. The ED₅₀ for **4** was 8 µmol/kg, documenting very potent anticonvulsant activity in this model of seizure, considerably higher than that of sodium valproate for which an ED₅₀ of 2.8 mM/kg in this paradigm was determined. Using a dose of 18 µmol/kg of **4,** activity was found at challenge times of 1, 2, and 4 h enabling the determination of a TPE, 2 h for 18 µmol/kg.

Results using the scMET seizure model revealed that doses of 5.7, 19, 57, 188, and 570 µmol **4** /kg were inactive at challenge times of 0.5 and 4 h with deaths occurring without seizure with a dose of 188 µmol/kg at the 4 h challenge time and a dose of 570 µmol/kg at the 0.5 challenge time. Oral treatment of rats with 94 µmol/kg was not protective with regard to MET-induced seizures at challenge times of 0.25, 0.5, 1, 2, and 4 h. However, these rats did experience continuous seizure activity, death following continuous seizure activity, or tonic seizure extension. These results are consistent with toxicities found in mice with higher doses of **4**.

Rotorod Toxicity was not observed when mice were treated ip with 5.7, 19, and 57 µmol/kg and challenged at 0.5 and 4 h. However, Rotorod Toxicity was observed with a larger dose of 188 µmol/kg at challenge times of 0.5 and 4 h with impaired ability to grasp the rotating rod and there were three deaths at the 4 h challenge time. Rotorod Toxicity was also observe with the 570 µmol/kg dose at the 0.5 and 4 h challenge times with impaired ability to grasp the rotating rod, sedation, and deaths without seizure, consistent with the high dose toxicities observed with other seizure models. Rats treated orally with 94 µmol/kg failed to evidence Rotorod Toxicity at 0.25, 0.5, 1, 2, and 4 h challenge times, again consistent with the absence of toxicity associated with the imposition of a CNS insult associated with the co-application of electroshock or Metrazol.

The µmolar level of anticonvulsant activity of Cu(Valp)₂Phen complex against MCS was not related to its individual constituents because the inorganic form of copper such as copper chloride or copper acetate, and the phenanthroline has no anticonvulsant of activity, and VPA has a much less potency, at the mmolar level, in this model of seizure.

Compound 4 was found is highly effective in preventing Minimal Clonic seizures, with a rapid onset and a prolonged duration of activity, at doses that do not cause Rotorod toxicity, a particularly useful pharmacological profile of activity for the treatment of Petit Mal seizures. Moreover, this protective effect, with an ED₅₀ 8µmol/kg, is largely superior to that of the parent ligand valproic acid. Such VPA-based metallo-complex which overcome undesired effects of the free ligand valproate will be useful in the future.

### 1.2.6 MTC and Anti-inflammatory results

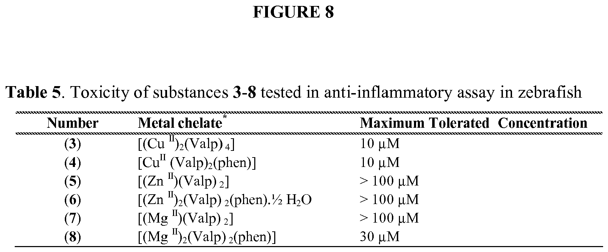

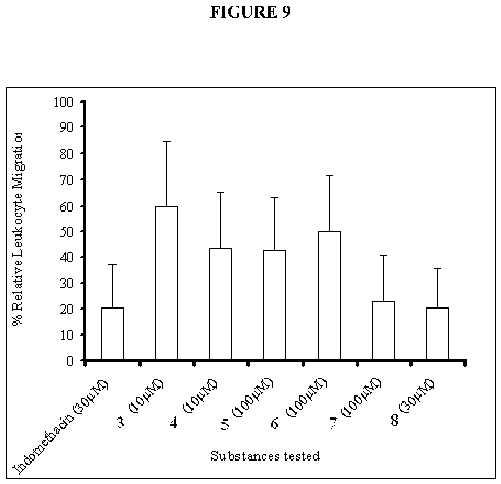

They are illustrated in table 5 of figure 8 and in figure 9.

Toxicity was not observed when zebrafish were treated with a 10 µM dose of each substance in the study. Compounds **5, 6** and **7** did not show any toxicity at 100 µM and were evaluated at this concentration. The other chemicals were evaluated at their respective MTC (figure 8).

Anti-inflammatory activity was evaluated following the steps described above. Leukocyte migration to damaged zone (end tail) was observed and evaluated as the parameter determining the anti-inflammatory activity of the tested compounds. Figure 9 reports the percentage relative of leukocyte's migration observed in zebrafish for each compound.

Only compound **3** showed a superior value (60%) while most of the complexes tested exhibited a percentage of relative leukocyte's migration inferior to 50% to dose used,. The magnesium derived complexes **5** and **6,** gave the best results with 23% and 20% relative leukocyte migration respectively, similar to the activity of the anti-inflammatory drug indomethacin as a positive control (20%). Compounds **3-8** are complexes of the anticonvulsant valproic acid that our theoretical model classified as inactive while the metallic corresponding copper, zinc or magnesium complexes were classified as active for at least 3 over our 13 theoretical models [Siverio-Mota, D., Iyarreta-Veitía, M., Dumas, F., Dioury, F., Ferroud, C., Cisneros, C. A., Matos, Herrera Pis, Y., Casañola Martin, G. M., Pérez-Giménez, F., Crawford, A. D., de Witte, P. and Marrero-Ponce,' Y., 2009, *in preparation*]*.* By comparing complexes **3/5** with **4/6,** magnesium(II) better than copper(II) could be responsible of the observed increased biological activity compared to the free valproate ligand.

Such an improvement in anti-inflammatory response of metal based drug versus the drug alone was observed for example in salicylates [Lemoine, P., Viossat, B., Dung, N. H., Tomas, A., Morgant, G., Greenaway, F. T., Sorenson, J. Inorg. Biochem. 2004, 98, 1734] and other non-steroidal anti-inflammatory drugs (NSAIDs) [J. R. Sorenson, J. Med. Chem. 19 (1976) 135-148.]. It was already reported that anti-inflammatory activity of flavonoids was increased when complexed with transition metals [Afanasév, I.B., et al., Biochem. Pharmacol., 2001. 61,677-684]. Indeed, naringin-derived Cu(II) complex has shown higher anti-inflammatory and anti-oxidant activity when compared with the free naringin [Hynes, M.J. and M. O'Coinceanainn,. J. Inorg. Biochem., 2004. 98, 1457 and Pereira, R.M.S., et al. Molecules, 2007. 12, 1352]. A very interesting result was observed for the magnesium based complexes, then the complexes [(Mg^{II})(Valp)₂] and [(Mg^{II})₂(Valp)₂phen)] presented the best result, opening the new horizons in the research for new anti-inflammatory lead compounds.

## Claims

1. A metal-based ternary complex of valproic acid with nitrogen donor ligands, in particular with diimines or diamines, wherein the metal in the complex is selected from the group consisting of Mg(II) and Zn(II) for its use in the treatment of a condition responsive to valproic acid therapy selected from cancer and inflammatory diseases.

2. A metal-based ternary complex of valproic acid with nitrogen donor ligands, for its use in the treatment of a condition responsive to valproic acid therapy selected from cancer and inflammatory diseases, according to claim 1, wherein the nitrogen donor ligand is a diimine selected from the group comprising 1,10-phenantroline, pyridine, imidazole, 1-methylimidazole, 2,9-dimethyl-1,10-phenantroline, phendione, 2,2'-bipyridine, 2,2'-bisbenzimidazole, thiabendazole (2-(4'-thiazolyl)-benzimidazole), 2-(4,5-dihydroxyoxazolin-2-yl)-1H-benzimidazole, 2-(2-pyridyl)-benzimidazole, 2-pyridylamine, 2-amino-2-thiazoline, nicotinamide, 2-picolinamide, 3-picoline, 4-picoline and dimethyldipicolinate.

3. A metal-based ternary complex of valproic acid with nitrogen donor ligands, for its use in the treatment of a condition responsive to valproic acid therapy selected from cancer and inflammatory diseases, according to claim 1, wherein the nitrogen donor ligand is a diamine selected in the group comprising ethylenediamine, 1,3-diaminopropane, N-methylethylenediamine, and N,N'-dimethylethylene-diamine.

4. A metal-based ternary complex of valproic acid with nitrogen donor ligands for its use in the treatment of a condition responsive to valproic acid therapy selected from cancer and inflammatory diseases according to claim 2, which is selected from the group comprising Mg(VALP)₂PHEN and Zn(VALP)₂PHEN.

5. A pharmaceutical composition comprising as active principle at least one metal-based ternary complex of valproic acid with nitrogen donor ligands, in particular with diimines or diamines, wherein the metal in the complex is selected from the group consisting of Mg(II) and Zn(II) associated with any pharmaceutical excipients for its use in the treatment of a condition responsive to valproic acid therapy selected from cancer and inflammatory diseases.

## Patentansprüche

1. Metallbasierter Ternärkomplex von Valpronsäure mit Stickstoffdonorliganden, insbesondere mit Diiminen oder Diaminen, wobei das Metall in dem Komplex ausgewählt ist aus der Gruppe, bestehend aus Mg(II) und Zn(II) für dessen Verwendung in der Behandlung eines Zustandes, der auf eine Valpronsäuretherapie anspricht, ausgewählt aus Krebs und Entzündungserkrankungen.

2. Metallbasierter Ternärkomplex von Valpronsäure mit Stickstoffdonorliganden, für dessen Verwendung bei der Behandlung eines Zustandes, der auf eine Valpronsäuretherapie anspricht, ausgewählt aus Krebs und Entzündungserkrankungen, nach Anspruch 1, wobei der Stickstoffdonorligand ein Diimin ist, das ausgewählt ist aus der Gruppe, umfassend 1,10-Phentantrolin, Pyridin, Imidazol, 1-Methylimidalzon, 2,9-Dimethyl-1,10-phentantrolin, Pyridin, Imidazol, 1-Methylimidazol, 2,9-Dimethyl-1,10-phenantrolin, Phendion, 2,2'-Bipyridin, 2,2'Bisbenzimidazol, Thiabendazol(2-)4'-Thiazolyl)-benzimidazol), 2-(4,5-Dihydroxyoxyzolin-2-yl)-1H-benzimidazol, 2-(2-Pyridyl)-Benzimidazol, 2-Pyridylamin, 2-Amino-2-thiazolin, Nicotinamid, 2-Picolinamid, 3-Picolin, 4-Picolin und Dimethyldipicolinat.

3. Metallbasierter Ternärkomplex von Valpronsäure mit Stickstoffdonorliganden, für dessen Verwendung bei der Behandlung eines Zustandes, der auf eine Valpronsäuretherapie anspricht, ausgewählt aus Krebs und Entzündungserkrankungen, nach Anspruch 1, wobei der Stickstoffdonorligand ein Diamin ist, das ausgewählt ist aus der Gruppe, umfassend Ethylendiamin, 1,3-Diaminopropan, N-Methylethylendiamin, und N,N'-Dimethylethylendiamin.

4. Metallbasierter Ternärkomplex von Valpronsäure mit Stickstoffdonorliganden, für dessen Verwendung bei der Behandlung eines Zustandes, der auf eine Valpronsäuretherapie anspricht, ausgewählt aus Krebs und Entzündungserkrankungen, nach Anspruch 2, der ausgewählt ist aus der Gruppe, umfassend Mg(VALP)₂PHEN und Zn(VALP)₂PHEN.

5. Pharmazeutische Zusammensetzung umfassend als einen aktiven Wirkstoff mindestens einen metallbasierten Ternärkomplex aus Valpronsäure mit Stickstoffdonorliganden, insbesondere mit Diiminen oder Diaminen, wobei das Metall in dem Komplex ausgewählt ist aus der Gruppe, bestehend aus Mg(II) und Zn(II) im Zusammenhang mit beliebigen pharmazeutischen Exzipienten für dessen Verwendung bei der Behandlung eines Zustandes, der auf eine Valpronsäuretherapie anspricht, ausgewählt aus Krebs und Entzündungskrankheiten.

## Revendications

1. Un complexe ternaire à base de métal d'acide valproïque avec des ligands donneurs d'azote, en particulier avec des diimines ou des diamines, dans lequel le métal du complexe est sélectionné dans le groupe constitué par le Mg(II) et le Zn(II) pour son utilisation dans le traitement d'une pathologie répondant à une thérapie à l'acide valproïque sélectionnée parmi le cancer et les maladies inflammatoires.

2. Un complexe ternaire à base de métal d'acide valproïque avec des ligands donneurs d'azote, pour son utilisation dans le traitement d'une pathologie répondant à une thérapie à l'acide valproïque sélectionnée parmi le cancer et les maladies inflammatoires, selon la revendication 1, dans lequel le ligand donneur d'azote est une diimine sélectionnée dans le groupe comprenant la 1,10-phénantroline, la pyridine, l'imidazole, le 1-méthylimidazole, la 2,9-diméthyl-1,10-phénantroline, la phendione, la 2,2'-bipyridine, le 2,2'-bisbenzimidazole, le thiabendazole (2-(4'-thiazolyl)-benzimidazole), le 2-(4,5-dihydroxyoxazolin-2-yl)-1-H-benzimidazole, le 2-(2-pyridyl)-benzimidazole, la 2-pyridylamine, la 2-amino-2-thiazoline, le nicotinamide, le 2-picolinamide, la 3-picoline, la 4-picoline et le diméthyldipicolinate.

3. Un complexe ternaire à base de métal d'acide valproïque avec des ligands donneurs d'azote, pour son utilisation dans le traitement d'une pathologie répondant à une thérapie à l'acide valproïque sélectionnée parmi le cancer et les maladies inflammatoires, selon la revendication 1, dans lequel le ligand donneur d'azote est une diamine sélectionnée dans le groupe comprenant l'éthylènediamine, le 1,3-diaminopropane, la N-méthylétlylènediamine, et la N,N'-diméthyléthylène-diamine.

4. Un complexe ternaire à base de métal d'acide valproïque avec des ligands donneurs d'azote pour son utilisation dans le traitement d'une pathologie répondant à une thérapie à l'acide valproïque sélectionnée parmi le cancer et les maladies inflammatoires, selon la revendication 2, qui est sélectionné dans le groupe comprenant le Mg(VALP)₂PHEN et le Zn(VALP)₂PHEN.

5. Une composition pharmaceutique comprenant comme principe actif au moins un complexe ternaire à base de métal d'acide valproïque avec des ligands donneurs d'azote, en particulier avec des diimines ou des diamines, dans lequel le métal du complexe est sélectionné dans le groupe constitué par le Mg(II) et le Zn(II) associé avec n'importe quel excipient pharmaceutique pour son utilisation dans le traitement d'une pathologie répondant à une thérapie à l'acide valproïque sélectionnée parmi le cancer et les maladies inflammatoires.
